# EUROPEAN PATENT APPLICATION

(11) **EP 2 243 824 A1**
(43) Date of publication of application: **27.10.2010**
(21) Application number: 08870727.8
(22) Date of filing: 17.12.2008
(51) Int. Cl.: C12M 1/34, G01N 27/02, G01N 33/48

(54) **BIOMASS CONCENTRATION MEASUREMENT DEVICE AND METHOD AND USE OF AN ELECTRONIC CHIP ELEMENT FOR MEASURING SAID BIOMASS CONCENTRATION**

(30) Priority: 16.01.2008 ES 200800109
(71) Applicant: Consejo Superior De Investigaciones Científicas, 28006 Madrid (ES); Universidad Autonoma De Barcelona, 08193 Bellaterra (Cerdanyola del Valles) Barcelona (ES)
(72) Inventor: MUÑOZ BERBEL, Xavier, E-08193 Bellaterra (Cerdanyola del Vallès) Barcelona (ES); MUÑOZ PASCUAL, Francisco Javier, E-08193 Bellaterra (Cerdanyola del Vallès) Barcelona (ES); ESCUDE PUJOL, Roger, E-08193 Bellaterra (Cerdanyola del Vallès) Barcelona (ES); MAS GORDI, Jordi, E-08193 Bellaterra (Cerdanyola del Vallès) Barcelona (ES); VIGUES FRANTZEN, Nuria, E-08193 Bellaterra (Cerdanyola del Vallès) Barcelona (ES); DEL CAMPO GARCÍA, Francisco Javier, E-08193 Bellaterra (Cerdanyola del Vallès) Barcelona (ES)
(74) Representative: Ponti Sales, Adelaida
(86) International application number: PCT/ES2008/070237
(87) International publication number: WO 2009/090280

(57) **Abstract**

The invention relates to a device for measuring the biomass concentration of a medium, including: means for measuring the change in electric impedance produced by the presence of biomass, and control and processing means for determining the biomass concentration from said means in order to measure the impedance change. The invention is **characterised in that** the impedance change measurement means include at least two electrodes having a suitable configuration to allow the measurement of the capacitance change of the double electric layer of the electrode/solution interface, produced by the electrostatic adhesion of the biomass. The invention is also **characterised in that** the processing and control means determine the biomass concentration from a calibration curve that correlates the capacitance change value with the biomass concentration. The invention also relates to the method used to measure the biomass concentration of the medium using impedance change measurement means. The invention further relates to the use of an electronic chip element for measuring biomass concentration.

## Description

### FIELD OF THE INVENTION

The present invention refers to a device and method for measuring the biomass concentration of the medium using measures of impedance change. It also refers to the use of an electronic chip element for measuring said biomass concentration.

### STATE OF THE ART

Biomass is an important control parameter in the field of fermentation, food industry, and clinical diagnosis, a proper monitoring of its activity being essential. These industrial areas feel the need of devices capable of differentiating and quantifying the microorganisms present in the medium.

The microbiological control of aqueous solutions in industrial fields such as the area of drinking and consumption water requires the quantification of very low microorganisms concentrations of 10² colonies forming units per cubic centimetres, from now onwards cfu.m⁻³.
The most commonly used methods up to now for measuring low bacterial biomass concentrations are those based on plate culture and PCR techniques. Plating is a simple and very reliable technique, but too slow (at least an incubation of 24 hours is needed).

The Impedance Electrochemical Spectroscopy (EIS) is a technique used in biomass characterization and quantification, which is based on the application of an electrical potential of variable frequency in the studied method, and in the measurement of the reply in current in the medium for every different frequency, using at least two electrodes. Said reply in current allows obtaining electrical impedance measured in an electrical circuit equivalent to the electrical model of the medium in study.
Frequency sweep gives raise to a spectrum of impedance in which, at a determined frequency, a decrease in the value of said impedance module caused by the changes produced in the solution is carried out by the products of the bacterial metabolism or by the cells in solution themselves. The value of the impedance change produced by the presence of biomass is used to determine the biomass concentration of the medium.

The existing impedimetric devices determining the biomass concentration using impedance electrochemical spectroscopy present the drawback of being slow, since long measurement times are required, especially for concentrations lower than 10³ cfu.cm⁻³. On the other hand, said devices also present the drawback of being unreliable for measuring low biomass concentrations, since the measure made is very sensitive to temperature and conductivity changes of the medium when the biomass is cultured. In low concentrations, the signal/noise ratio is very small, so measures cannot be correctly made.

Patent ES 2143911 describes a method an apparatus for measuring biomass concentration aimed at improving the reliability and quickness of existing methods and devices, mostly regarding of low biomass concentrations measurements.

The method of the mentioned patent estimates the biomass concentration from the relationship between impedance at high and low frequency. In a first process option, the impedance values obtained at high and low frequencies are directly used and, in a second option, two values resulting from adjusting the impedance data of a frequency swept in a curve are used. In the method used, a circuit equivalent to the electrical model of the medium, which composed of resistance of the extracellular medium, resistance of the intracellular medium and capacitance of cell membrane, is accepted by convention.

The impedimetric device described in the abovementioned patent comprises reading probes designed to adapt themselves to two standard connections of an industrial bioreactor, which include a temperature and conductivity sensor of the medium and a set of electrodes. Electrodes of each probe are placed two by two on the longitudinal edges of a transverse oblong orifice, or in another embodiment, on the plate or support in which the thin layer of culture is placed. In both cases the electrodes configuration has been designed on the one to limit the electric field in a defined way, facilitating the biomass measurement at low concentrations, and on the other hand, to reduce the possibility of having bubbles present in the biomass that could affect the measurement.

However, the device and method described in the mentioned patent still has limitations, since the moderated variations experienced by the conductivity of the extracellular medium (conductivity of the solution) in the culture still can disguise the measurement of low biomass concentrations.

Patent ES2183677 constitutes an improvement of the abovementioned patent, aimed at improving the reliability of the detection method at low biomass concentrations. The proposed improved method includes the measurement of the medium electric impedance in two areas, one without biomass and another with biomass. Measurement is carried out in both areas using sets of identical electrodes in order to gain information about the conductivity variations of the medium in the area without biomass. The obtained information is used to correct the estimation of biomass concentration of the medium in the area with biomass.

However, despite the proposed improvements, the measurement method of the abovementioned patent is complex and also ineffective, because it keeps on presenting limitations when measuring biomass concentrations lower than 10⁵ cfu.cm⁻³.

### DESCRIPTION OF THE INVENTION

The objective of the present invention is to solve the mentioned drawbacks by developing a device and method for measuring low biomass concentrations that present the advantage of being quick, simple and reliable.

Following this objective, according to a first aspect, the present invention provides a device for measuring biomass concentration in a medium which includes means for measuring the change in electric impedance produced by the presence of biomass and processing and control means for determining the impedance change. The device is **characterized in that** the impedance change measuring means comprise at least two electrodes having a suitable configuration to allow the measurement of the capacitance change of the electrical double layer of the electrode/solution interface produced by electrostatic adhesion of biomass, and in that the processing and control means determine the biomass concentration from a calibration curve that correlates said capacitance change with the biomass concentration.

Following the same objective, according to a second aspect, the present invention provides a method for measuring the biomass concentration of a medium using the claimed device, **characterized in that** it includes the steps of:
a) polarizing the medium by applying an electric potential to the electrodes of the claimed device, in order to adhere biomass on the surface of said electrodes,
b) measuring the change in electric impedance of the polarized medium,
c) determining the value of the capacitance change of the electrical double layer of the electrode/solution interface (Cdl) from the impedance value change of step b), said value of the capacitance change being determined in the equivalent circuit to the medium electrical model,
d) determining the medium biomass concentration from the value of capacitance change of the electrical double layer (Cdl) using the corresponding calibration curve which correlates said value of capacitance variation with the biomass concentration of the medium.

Lastly, according to a third aspect, the present invention refers to the use of an electronic chip element including at least two electrodes integrated in a material substrate for measuring a medium biomass concentration using impedance electrochemical spectroscopy.

The claimed device and method are based on the measurement of the changes occurred in the electrode/solution interface due to the adhesion of biomass.

The experiments done so far have permitted observing that the biomass adhered to the surface of the working electrode during the first colonization minutes modifies the electrochemical characteristics of the double electric layer of the electrode interface. The detected change depends on the amount of adhered biomass, which at the same time, is proportional to the biomass concentration of said biomass in the medium. This fact has made possible the development of a device and method for quantifying the biomass concentration, prior calibration of the device in the medium, temperature and biomass of interest, which has the advantage of being quick, direct and simple.

The double electric layer of the solution/electrode interface is commonly considered as similar to an electric condenser, so the changes produced in this layer can be easily monitored by impedance electrochemical spectroscopy.

The experiments done have allowed observing the biomass adhered to the surface of the working electrode, which at determined working frequencies, behaves as a dielectric material that modifies the capacitance value of the double electric layer (Cdl) of the device electrodes. The capacitance variation of the double layer (Cdl) has been seen as dependent of the medium biomass concentration, that is why it is has been appreciated that monitoring the capacitance changes of that layer allows the detection of the biomass concentration of the medium in a simple and reliable way, using the corresponding calibration curve.

In the claimed device, the configuration of electrodes (in particular, their area and distance between them) should be suitable to allow the measurement of the capacitance change of the electrode/solution interface produced by the biomass adhesion. This is to say, it should be appropriate to allow limiting the electric field to the interface area which is the area where the electrochemical changes produced by the presence of biomass adhered to the working electrode are measured.

Surprisingly, it has been found that as the proposed method is based on a measurement on the electrode interface, medium characteristics such as conductivity do not significantly affect the measurements.

Concretely it has been found that whereas the double layer capacitance increases continuously with the biomass concentration, the solution resistance, which informs about the average conductivity of the medium, keeps constant with the increase of biomass concentration. Therefore, surprisingly the value of capacitance change of the interface is not modified by the conductivity change of the medium during the culture phase, constituting a very reliable parameter for measuring the biomass concentration. Thanks to this, the claimed device and method present the advantage of allowing the detection of very low biomass concentrations, of until 10² cfu.cm-³, in a very reliable way.

Another advantage of the claimed device and method is the quickness when measuring the concentration. This is because it has been found that the change of impedance signal produced by the biomass adhesion can be measured in a very short exposure time, approximately very few minutes.

Once calibrated, the device allows determining the biomass concentration from a sole impedance measurement, as well as monitoring said biomass concentration during a medium incubation period. After every measurement, it will be necessary to carry out the cleaning of the electrodes surface and, whenever needed, recalibrating the device.

In the present invention, biomass is generally understood as biological material constituted by one or more prokaryotic or eukaryotic cells and particularly, microorganisms as yeasts, fungus, etc. Said biomass will be provided suspended in any type of medium, although preferably the medium to be used will be an aqueous solution or medium.

The size of the device electrodes affects the sensibility of the measurement; in this sense, electrodes will preferably be microelectrodes. However, optionally it has been foreseen that electrodes could be nanoelectrodes in order to improve even more said sensibility.

According to a preferred embodiment, the device electrodes are integrated in a substrate material, said electrodes integrated in said substrate constituting an electronic element of reduced size or electronic chip element capable of being immersed in the medium in which the biomass concentration measurement is carried out.

Thanks to this, the biomass measurement can be carried out by an electronic chip element which, thanks to its reduce size, allows making the measurements in very small sample volumes, and using very short measurements times, lower than a minute.

The chip or electronic element of reduced size provides a very versatile biomass sensor device to be applied in a wide diversity of industrial fields: food, clinical diagnosis, waters, etc., in which microbiological control of the medium is needed.

Advantageously said electronic chip element includes means of wireless connection to the processing and control means of the device. In this way, the device results even more versatile, adaptable to a higher number of situations.

Again advantageously, the electronic chip element has, in the same substrate or in a close substrate, at least an integrated sensor for measuring the medium in which the measurement of biomass concentration is carried out. Said sensor can be selected from a group consisting of flow, temperature, conductivity, pH, oxygen sensors or can be for example a biocide selective sensor.

The addition of one or more sensors useful for measuring different medium parameters leads to increase even more the versatility of the device of the present invention.

According to an embodiment, electrodes are integrated in the chip substrate as microdiscs. This geometry results very simple and efficient. However, the geometry of the integrated electrodes can be very varied, as the number of electrodes. In the same way, the material of the electrodes can be varied, conductor o semiconductor type. In the latter case, the device will be especially suitable for minimizing the corrosion phenomena of certain media.

In reference to the method of the present invention, according to a preferred embodiment of said method, the capacitance of the double electric layer (Cdl) of the equivalent circuit on which the electrical impedance is measured, is approximate to an element of constant phase (CPEdl), the value of the module variation (Kdl) of said element of constant phase being determined in step c).

The element of constant phase takes into account the loss of ideal capacitative behaviour of the double electric layer of the interface due to the superficial roughness of the electrodes, being a very useful parameter that improves the adjustment of the calibration curve used in the method.

Experiments done show that the module variation of the element of constant phase (Kdl) presents a high quality lineal relation with the presence of biomass in a wide range of concentrations, therefore being a very suitable parameter for measuring biomass concentration using the method of the present invention.

According to another embodiment of the present invention, the biomass concentration of step d) is determined from the differential value of two capacitance change measurements (Cdl) measured in a predetermined period of time.

Experiments that have been carried out show that the variation of the differential of the double layer capacitance change presents a lineal relation with the biomass concentration in a higher range of concentrations. In this way, an even wider range of biomass concentrations, including very low values, in the range of 10¹ cfu.cm⁻³, until higher values, higher than 10⁸ cfu.cm⁻³, can be measured.

As it has been commented before, characteristics of the medium, such as conductivity changes, do not significantly affect the measurement. However, it has been found that low salinity media with low conductivity are preferable, since the biomass detection limit is lower in these media.

The range of working frequencies of the electrical flow generated by the measurement of impedance in step b) can change depending on the configuration and material of the electrodes used. However, preferably, frequencies lower than 1 MHz will be used, particularly frequencies of KHz, since said frequencies have been shown to be more suitable for measuring the capacitance of the electrode/solution interface.

Anyway, the frequencies used will be those suitable for facilitating the behaviour of adhered biomass as a dielectric material that modifies the capacitance value of the double layer (Cdl). In this sense, those working frequencies in which the current flow generated goes through the cell walls are to be avoided.

Preferably, the time used to carry out at least steps a) and b) of the method will be lower than 1 minute. It has been found that this measurement time improves the correlation obtained between capacitance and biomass concentration.

According to an embodiment, the initial biomass concentration of the medium is indirectly determined by the corresponding growing curve of said biomass, after monitoring the biomass concentration during a determined incubation period with the device of the present invention. In this way, it is possible to indirectly detect medium biomass concentrations even lower than 10² cfu.cm⁻³.

### BRIEF DESCRIPTION OF THE DRAWINGS

For a better understanding of what has been exposed, the attached drawings represent several practical cases of embodiments, schematically and only as a non-limitative example.
In those drawings,
Figure 1 is a schematic plan view of the structure of a first type of small electronic element or electronic chip element used for measuring biomass concentration.
Figure 2 is a schematic plan view of the structure of a second type of electronic chip element used for measuring biomass concentration.
Figures 3a and 3b are two schematic representations of a working electrode of the device, after polarization.
Figures 4a and 4b are two schematic representations of the same working electrode of figure 3, showing said electrode at the beginning of the biomass adhesion process, time in which the measurement is carried out.
Figures 5a and 5b are two schematic representations of the same working electrode in figure 3, after a long exposition to the solution with biomass.
Figure 6 is a graphical representation showing the capacitance variation of the double electric layer with the bacterial biomass concentration of the medium, in two media of different conductivity.
Figure 7 is a graphical representation showing the solution resistance variation (measured in the interface) with bacterial biomass concentration, in two media of different conductivity.
Figure 8 is a graphical representation showing the variation of the differential of the capacitance change with the biomass concentration, in measurements taken in a 40-minutes period.

### DESCRIPTION OF EXAMPLES AND PREFERRED EMBODIMENTS

As it has been noted in the description of the invention, in a preferred embodiment of the device of the present invention, the measurement of electrical impedance is carried out by a chip 1 or electronic element of reduced size, which has the integrated electrodes 2 with which the measurement of electrical impedance is made.

As it has been noted, chip 1 is an electronic element of reduced size having the advantage of allowing the realization in a very comfortable way of the measurements of biomass concentrations in very small sample volumes.

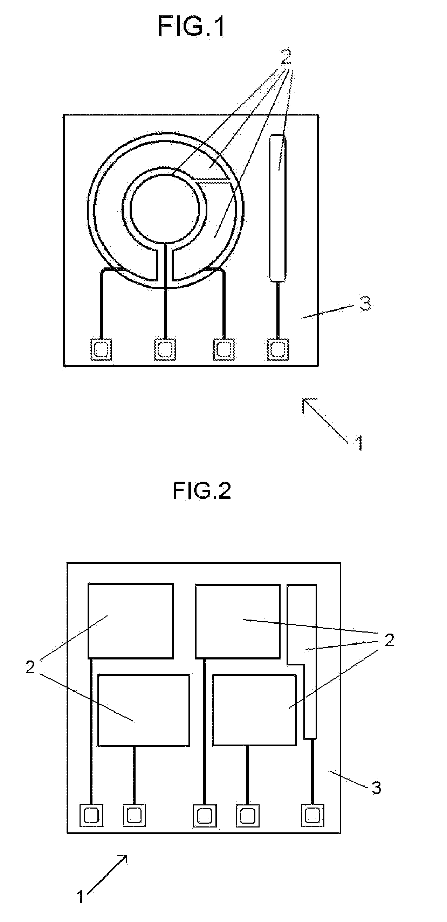

Figure 1 shows one of the types of chip 1 used in the present invention, which has in the same substrate 2 two micro-electrodes 2 integrated in the shape of microdiscs; auxiliary electrode and working electrode, and a third electrode 2 of reference of different geometry. Figure 2 shows a second type of chip 1 including in this case an amount of five electrodes 2.

The material of the substrate 3 and of the chip 1 electrodes 2 can be very varied, no limitation having been found at this respect. Thus, within the material tested for the substrate 3, glass and quartz are mentioned. The materials mentioned for electrodes 2 are conductive materials such as gold, platinum, carbon, iridium, etc., However, also semiconductive materials can be used, such as oxides and nitrides, which have the advantage of minimizing the corrosion phenomena caused by the medium.

As it has been noted in the description of the invention, the claimed device and method are based on the measurement of the changes produced in the electrode/solution interface due to the adhesion of biomass.

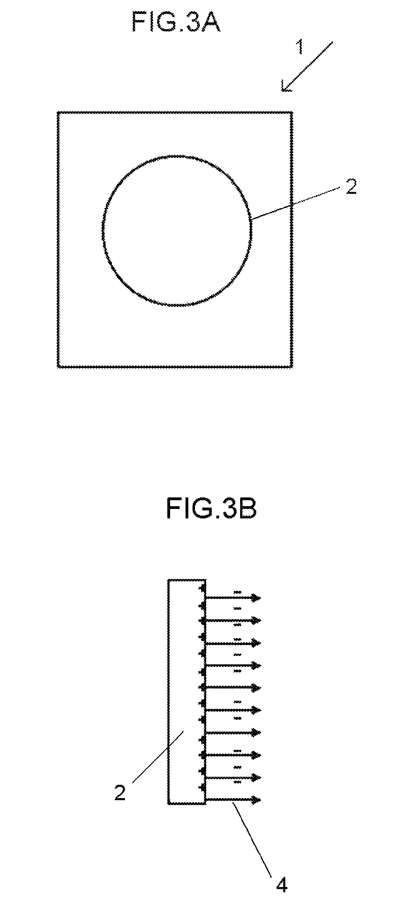

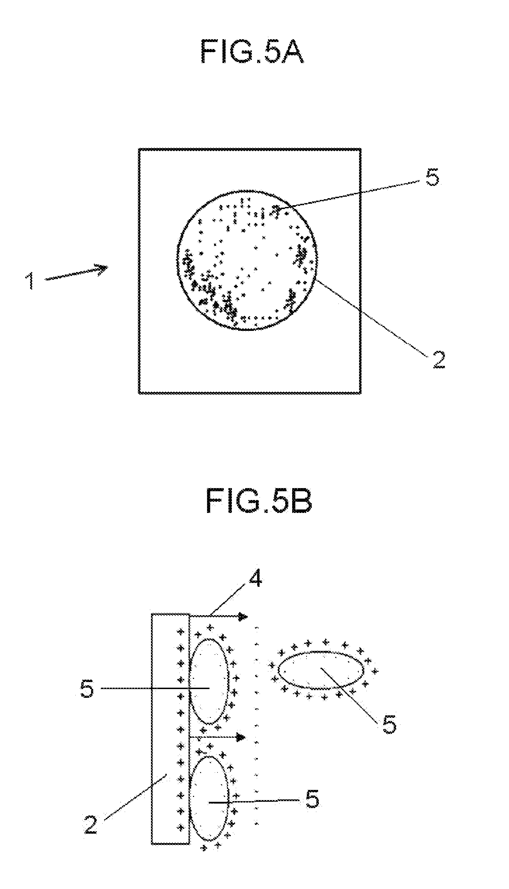

Figures 3 to 5 show a schematic view of the changes produced on the chip 1 surface when the measurement of biomass concentration is being carried out.

In the first step of the process shown by figures 3a and 3b, an electrical potential is applied to the electrodes 2, which is translated into a separation of charges leading to the appearance of the double electric layer 4 in the electrode/solution interface. Said separation of charges attracts the biomass 5 being suspended in the medium, which quickly adheres to the surface of the chip 1 working electrode 2.

The applied electrical potential can change depending on the medium. However, it has been found that the lowest applied potentials are translated into a lower electrode 2 area covered by biomass 5, thereby facilitating the measurement in high medium concentrations. The sensibility of the method changes with the applied potential, the lower the potential, the lower the slope of the calibration curve, and therefore, the lower the process sensibility.

During the first steps of biomass 5 colonization, a dynamic equilibrium between the adhered cells and the free cells which physically and electrochemically modify the double electric layer 4 of the working electrode 2 interface is established.

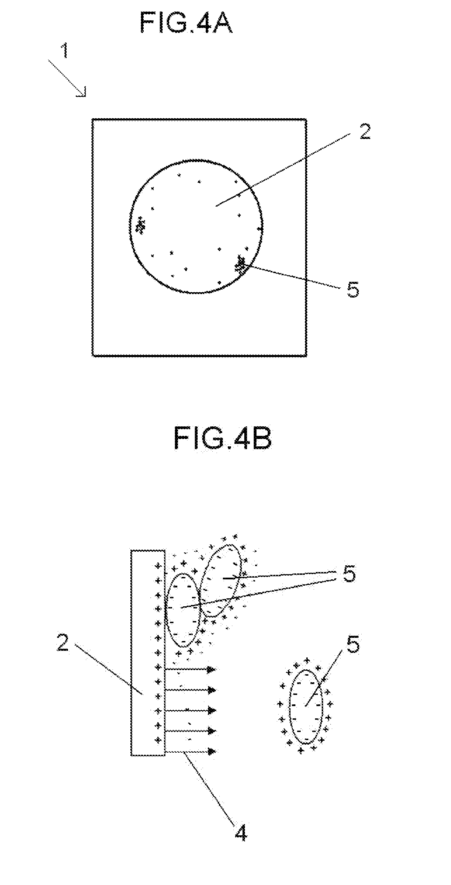

Figures 4a and 4b show the state of the double electric layer 4 the time in which the measurement of the process change in electric impedance is carried out.

The impedance measurement is carried out by impedance electrochemical spectroscopy, in a range of frequencies lower than 1 MHz, preferably 1 KHz. In this electrical environment the bacterial biomass adhered to the chip 1 surface behaves like a dielectric material modifying the capacitance value of the double electric layer (Cdl) of the device working microelectrode 2.

The variation in the capacitance change is monitored by impedance electrochemical spectroscopy, setting the impedance spectra to a circuit composed of, besides the double layer (Cdl) capacitance, the solution resistance (Rs) and the reference electrode capacitance (Celec), if used in the measurement.

The medium biomass concentration is determined from the capacitance change (Cdl) value, through a calibration curve that correlates the double layer (Cdl) capacitance change value with the biomass concentration in the working range.

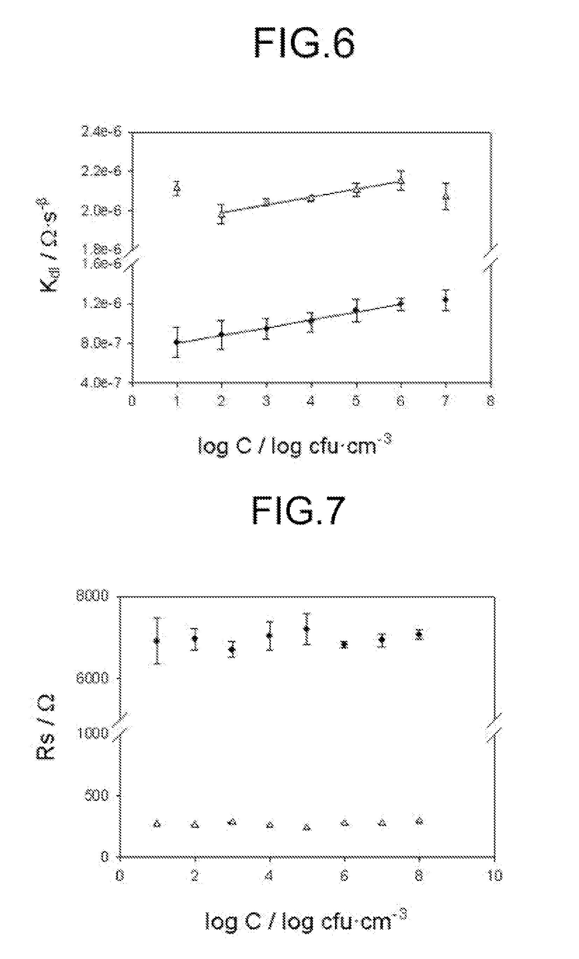

Figure 6 shows a graphical representation of the capacitance variation of the double electric layer (4) with the biomass (5) concentration of the medium, in two different conductivity media. In that figure, the capacitance value has been adjusted to the value of the element of constant phase (Kdl), since it has been experimentally found that said module variation presents a high quality relation in a wide range of concentrations with the biomass concentration.

Figure 7 is a graphic representation showing the solution resistance variation (measured in the interface) with the bacterial biomass concentration, in both different conductivity media used in figure 6.

The results shown in figures 6 and 7 have been obtained by experiments carried out with samples of bacterial biomass suspended in an aqueous solution of sterile serum.

As can be observed in said figures 6 and 7, the module variation of the phase element (Kdl) shows a continuous increase with the biomass concentration in both media of different conductivities. However, the solution resistance (Rs) of both media, which informs about the average conductivity, keeps constant along the concentrations range. From these results it can be assumed that the capacitance value is not modified by a local conductivity change of the medium due to the biomass increase, but it does by an increase in the bacterial concentration of the sample, obtaining a high quality lineal relation between the capacitance change value and the biomass concentration, ranging from 10¹ cfu.cm⁻³ to 10⁶ cfu.cm⁻³.

The obtained results show that the capacitance change value of the interface double electric layer (4) is a useful a very reliable parameter for measuring low biomass (5) concentrations.

From figures 6 and 7, it can also be assumed that the extent of the interface capacitance change varies depending on the type of conductivity of the medium. Thus, in the same bacterial concentration at low medium conductivities, lower capacitance change (Kdl) values were showed.

The conductivity difference of the starting medium does not imply an improvement in the process sensibility, which is identical in both cases (same curve slope). However, the decrease in the working medium conductivity has been observed to improve the detection limit, which is reduced from 10² cfu.cm-3 until 10¹ cfu.cm-3. Therefore, the low salinity media result more suitable for measuring low biomass concentrations.

As it has been noted in the description of the invention, in order to measure a wider biomass concentrations range, a parameter measuring the capacitance value difference of two measurements taken in a determined period of time has been defined.

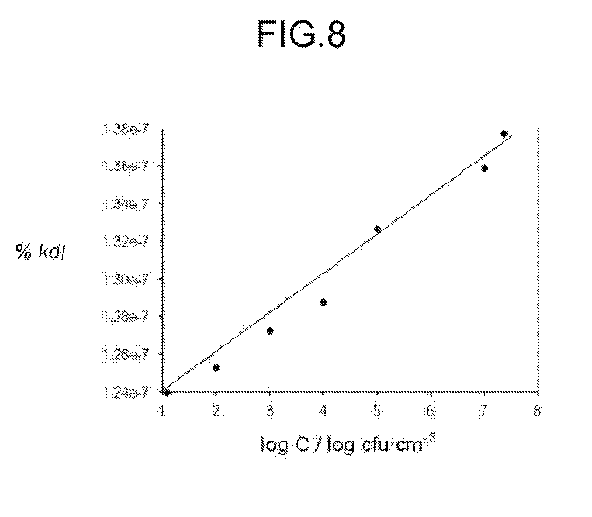

Figure 8 shows the graphical representation of the results obtained in an experiment in which the variation of the differential of the capacitance change with the bacterial biomass concentration was measured in measurements taken in a 40-minutes period.

Results show a high quality lineal relation in a concentration range from 10 cfu.cm⁻³ to 108 cfu.cm⁻³.

## Claims

1. Device for measuring the biomass (5) concentration of a medium, comprising means for measuring the change in electric impedance produced by the presence of biomass (5) and processing and control means for determining the biomass (5) concentration from these means for measuring the impedance change, **characterized in that** those means for measuring the impedance change comprise at least two electrodes (2) having a suitable configuration to allow measuring the change of capacitance of the electrical double layer (4) of the electrode/solution interface produced by biomass (5) electrostatic adhesion, and **in that** said processing and control means determine the biomass (5) concentration from a calibration curve that correlates said value of capacitance change with the biomass (5) concentration.

2. Device according to claim 1, **characterized in that** said at least two electrodes (2) are integrated in a material substrate (3), said electrodes (2) integrated in said substrate constituting an electronic chip element (1) capable of being immersed in the medium in which the biomass (5) concentration measurement is carried out.

3. Device according to claim 2, **characterized in that** said electronic chip element (1) comprises means of wireless connection to said processing and control means.

4. Device according to claim 2, **characterized in that** said electronic chip element (1) comprises at least a sensor for measuring parameters of the medium in which the biomass (5) concentration measurement is carried out.

5. Device according to claim 2, **characterized in that** said electrodes (2) are integrated in the substrate (2) in form of microdiscs.

6. Device according to claim 1, **characterized in that** the material of said electrodes (2) is a semiconductor material.

7. Device according to claim 1, **characterized in that** said biomass (5) is constituted by one or more prokaryotic or eukaryotic cells.

8. Use of an electronic chip element (1) comprising at least two electrodes (2) integrated in the same material substrate (3) for measuring the biomass (5) concentration of a medium using impedance electrochemical spectroscopy.

9. Method for measuring the biomass concentration of a medium using the claimed device, **characterized in that** it comprises the steps of:
a) polarizing the medium by applying an electrical potential to the electrodes (2) of the claimed device, in order to adhere biomass on the surface of said electrodes (2),
b) measuring the electric impedance change of the polarized method,
c) determining the value of the capacitance change of the electrical double layer (4) of the electrode/solution interface from the value of impedance change of step b), said value of the capacitance change being determined in the equivalent circuit to the medium electrical model,
d) determining the biomass (5) concentration of the medium from the value of capacitance change of the electrical double layer (4) using the corresponding calibration curve which correlates said value of capacitance change with the biomass (5) concentration of the medium.

10. Method according to claim 9, **characterized in that** the capacitance of the double electrical layer (4) of the equivalent circuit is approximate to an element of constant phase, the value of the module variation of said element of constant phase being determined in step c).

11. Method according to claim 9, **characterized in that** the working frequency of the electric flow generated by the impedance measurement in step b) is lower than 1 MHz.

12. Method according to claim 9, **characterized in that** the biomass concentration of step d) is determined from the differential value of two capacitance change measurements taken in a determined period of time.

13. Method according to claim 9, **characterized in that** the initial biomass (5) concentration of the medium is indirectly determined, using the corresponding biomass (5) growing curve after monitoring the biomass (5) concentration during a determined incubation period.

14. Method according to claim 9, **characterized in that** said biomass (5) is constituted by one or more eukaryotic or eukaryotic cells.
